# EUROPÄISCHE PATENTSCHRIFT

(11) **EP 0 646 123 B1**
(45) Veröffentlichungstag und Bekanntmachung des Hinweises auf die Patenterteilung: **16.04.1997**
(21) Anmeldenummer: 93912773.4
(22) Anmeldetag: 24.05.1993
(51) Int. Cl.: C07H 15/04

(54) **VERFAHREN ZUR HERSTELLUNG HELLFARBIGER TENSIDE MIT VERMINDERTEM PEROXIDGEHALT**
METHOD OF PRODUCING PALE-COLOURED SURFACTANTS WITH A REDUCED PEROXIDE CONTENT
PROCEDE DE PREPARATION D'AGENTS TENSIOACTIFS DE COULEUR CLAIRE A TENEUR REDUITE EN PEROXYDES

(30) Priorität: 01.06.1992 DE 4218073
(43) Veröffentlichungstag der Anmeldung: 05.04.1995
(73) Patentinhaber: Henkel Kommanditgesellschaft auf Aktien, 40191 Düsseldorf (DE)
(72) Erfinder: WEUTHEN, Manfred, D-5650 Solingen 11 (DE)
(86) Internationale Anmeldenummer: EP9301299
(87) Internationale Veröffentlichungsnummer: WO9428005

(56) Entgegenhaltungen:
- DE-A- 3 319 591
- PATENT ABSTRACTS OF JAPAN Vol. 15, no. 017 (C-0796) 5. April 1989
- DATABASE WPI Section Ch, Week 8751, Derwent Publications Ltd., London, GB; Class D25, AN 87-358813

## Beschreibung

### Gebiet der Erfindung

Die Erfindung betrifft ein Verfahren zur Herstellung hellfarbiger Tenside mit vermindertem Peroxidgehalt, bei dem man Tensidpasten in Gegenwart ausgesuchter Übergangsmetallverbindungen mit Wasserstoffperoxid behandelt.

### Stand der Technik

Bei der Herstellung einer Vielzahl von Tensiden kommt es zu einer unerwünschten Verfärbung, die zwar in der Regel die Produktqualität nicht beeinträchtigt, jedoch eine Vermarktung aus ästhetischen Gründen erschwert oder sogar unmöglich macht. Die Ursachen hierfür können vielfältiger Natur sein: bei anionischen Tensiden, die über den Weg der Sulfierung hergestellt worden sind, handelt es sich in der Regel um Oxidationsnebenprodukte des verwendeten Schwefeltrioxids, während Verfärbungen beispielsweise in Alkyloligoglycosid-Pasten häufig auf einen geringen Anteil an Verkohlungsprodukten des Glycosidkörpers zurückgeführt werden können.

Zur Verbesserung der Farbqualität werden Tenside bzw. Tensidpasten daher üblicherweise einem Bleichprozeß unterworfen, wobei vorzugsweise Wasserstoffperoxid Anwendung findet (vgl. Z.B. JP-A 62/260894 oder E.Woollatt in "The manufacture of soap ...", (1985), S.384-385).

Bei der Verwendung von Wasserstoffperoxid als Bleichmittel muß jedoch dafür Sorge getragen werden, daß der Restgehalt an Peroxiden in den gebleichten Tensidpasten möglichst gering ist und vorzugsweise unterhalb von 1 ppm liegt. Anderenfalls besteht die Gefahr, daß die Peroxide in den Endformulierungen mit weiteren Inhaltsstoffen, wie beispielsweise Farbstoffen, Duftstoffen oder UV-Filtern, abreagieren können oder sich in den Transportbehältern durch Freisetzung von Sauerstoff ein Druck entwickelt.

Um dies sicher auszuschließen, werden nicht abreagierte Peroxidverbindungen in gebleichten Tensidpsten üblicherweise thermisch zerstört. Dies führt jedoch wiederum zu einer oxidativen und thermischen Belastung des Produktes, die einen Teil der zuvor erzielten Bleichwirkung zunichte machen kann.

Aus der Offenlegungsschrift **EP-A-0 387 913** (Kao) ist bekannt, daß Peroxidverbindungen auch mit Hilfe von Reduktionsmitteln wie beispielsweise Natriumborhydrid zerstört werden können. Ein solches Verfahren erfordert jedoch wiederum eine anschließende Zerstörung nichtumgesetzten Borhydrids mit Säure und führt zu einer vielfach unerwünschten Belastung der Produkte mit Salzen und Borverbindungen.

Gemäß der Lehre der **JP-A H2/264789** lassen sich hellfarbige Alkylpolyglucoside herstellen, indem man die Peroxidbleiche im alkalischen Medium in Gegenwart beispielsweise von Braunstein oder Platin durchführt.

In der Offenlegungsschrift **EP-A-0 338 151** (Henkel) wird vorgeschlagen, wäßrige Pasten von Alkyloligoglycosiden nach der Bleiche einer hydrierenden Nachbehandlung in Gegenwart von Edelmetallkatalysatoren zu unterwerfen. Ein solches Verfahren erfordert jedoch einen hohen technischen Aufwand und kommt für eine Realisierung daher nur eingeschränkt in Betracht.

Die Aufgabe der Erfindung bestand somit darin, ein verbessertes Verfahren zur Herstellung hellfarbiger Tenside mit vermindertem Peroxidgehalt zur Verfügung zu stellen, das frei von den geschilderten Nachteilen ist.

### Beschreibung der Erfindung

Gegenstand der Erfindung ist ein Verfahren zur Herstellung hellfarbiger Tenside mit vermindertem Peroxidgehalt, bei dem man Pasten anionischer, nichtionischer undloder amphoterer bzw. zwitterionischer Tenside in Gegenwart von Platinmetallverbindungen mit Wasserstoffperoxid behandelt, das sich dadurch auszeichnet, daß man als Platinmetallverbindung Palladium bzw. Platin auf Aktivkohle in Mengen von 10 bis 1000 ppm - bezogen auf den Feststoffgehalt der Pasten - einsetzt.

Überraschenderweise wurde gefunden, daß schon kleine Mengen von Platin bzw. Palladium auf Aktivkohle den Abbau von Peroxiden in wäßrigen und/oder alkoholischen Tensidpasten signifikant beschleunigen. Durch Zusatz dieser Katalysatoren während oder unmittelbar nach der Bleiche werden Tensidpasten erhalten, deren Peroxidgehalt zuverlässig unterhalb von 10, insbesondere bei 1 bis 5 ppm - bezogen auf den Feststoffgehalt der Paste - liegt.

Das erfindungsgemäße Verfahren ist auf die Verminderung des Peroxidgehalts in Tensidpasten gerichtet. Hierbei ist der Feststoffgehalt der Pasten an sich unkritisch und nur insofern von Bedeutung, daß die Anwendung auf stark verdünnter Tensidlösungen wirtschaftlich unrentabel ist, während hochkonzentrierte Tensidpasten häufig eine solch hohe Viskosität aufweisen, daß das Einbringen der Komponenten mechanisch nicht oder nur unter großen Schwierigkeiten möglich ist. Vorzugsweise kommen daher Tensidpasten zum Einsatz, die einen Feststoffgehalt von 5 bis 85, vorzugsweise 25 bis 70 Gew.-% aufweisen.

Im Sinne des erfindungsgemäßen Verfahrens können Pasten anionischer, nichtionischer und/oder amphoterer bzw. zwitterionischer Tenside bereitgestellt werden, die sich durch ausgezeichnete Farbqualität und einen niedrigen Restgehalt an Peroxiden auszeichnen.

Typische Beispiele für **anionische Tenside** sind Alkylbenzolsulfonate, Alkansulfonate, Paraffinsulfonate, Olefinsulfonate, Alkylethersulfonate, Glycerinethersulfonate, α-Methylestersulfonate, Sulfofettsäuren, Alkylsulfonate, Fettalkoholethersulfate, Glycerinethersulfate, Monoglycerid(ether)sulfate, Fettsäureamid(ether)sulfate, Hydroxymischethersulfate, Sulfosuccinate, Sulfosuccinamate, Sulfotriglyceride, Isethionate, Tauride, Sarcosinate, Ethercarbonsäuren und Alkyl(ether)phosphate.

Als **nichtionische Tenside** kommen beispielsweise Saccharoseester, Sorbitanester und Polysorbate in Betracht; als besonders wirksam hat sich das Verfahren bei Anwendung auf Pasten von Alkyloligoglycosiden, insbesondere Alkyloligoglucosiden erwiesen.

Typische Beispiele für **amphotere bzw. zwitterionische Tenside** stellen Alkylamidobetaine, Aminopropionate, Imidazoliniumbetaine und Sulfobetaine dar.

Die Bleiche der Tensidpsten kann mit 0,1 bis 5, vorzugsweise 1 bis 3 Gew.-% Wasserstoffperoxid - bezogen auf den Feststoffgehalt der Paste - durchgeführt werden. Üblicherweise wird das H₂O₂ hierzu in Form einer verdünnten, beispielsweise 30 gew.-%igen Lösung eingesetzt.

### Gewerbliche Anwendbarkeit

Die nach dem erfindungsgemäßen Verfahren erhältlichen Tensidpasten sind hellfarbig und weisen einen niedrigen Peroxidgehalt auf. Sie eignen sich zur Herstellung von Wasch-, Spül- und Reinigungsmitteln sowie Produkten der Haar- und Körperpflege, in denen sie in Mengen von 1 bis 80, vorzugsweise 10 bis 50 Gew.-% - bezogen auf die Mittel - enthalten sein können.

Die nachfolgenden Beispiele sollen den Gegenstand der Erfindung näher erläutern, ohne ihn darauf einzuschränken.

### Beispiel 1 und Vergleides beispiele V1 - V6

**Allgemeine Versuchsvorschrift.** Eine ungebleichte wäßrige Paste eines technischen C₁₂/₁₄-Kokosalkylglucosids (Plantaren^{(R)} APG 600, Feststoffgehalt 50 Gew.-%, Fa. Henkel KGaA, Düsseldorf, FRG) wurde mit 25 gew.-%iger wäßriger Natriumhydroxidlösung auf einen pH-Wert von 13,5 eingestellt. Anschließend wurde die Paste mit 2 Gew.-% - bezogen auf den Feststoffanteil - Wasserstoffperoxid in Form einer 30 gew.%igen wäßrigen Lösung sowie gegebenenfalls 10 bis 100 ppm einer Übergangsmetallverbindung - jeweils bezogen auf den Feststoffgehalt der Paste - versetzt. Die Paste wurde über einen Zeitraum von 30 min auf 90 °C erhitzt und die Peroxidwerte nach 5, 10 und 30 min. bestimmt. Die Ergebnisse sind in Tab. 1 zusammengefaßt.

**Tab. 1:**

| Peroxid-Abbau | | | | | |
|---|---|---|---|---|---|
| Bsp. | Katalysator | c(Kat) ppm | POZ nach | | |
| | | | 5 min | 10 min | 30 min |
| V1 | ohne | - | 280 | 260 | 190 |
| V2 | MnCl₂ | 10 | 18 | 4 | <1 |
| V3 | CnCl₂ | 10 | 45 | 8 | <1 |
| V4 | FeSO₄ | 100 | 101 | 36 | 1 |
| V5 | CuO | 1000 | <1 | - | - |
| V6 | PdCl₂ | 10 | 7 | 1 | <1 |
| 1 | Pt/A-Kohle | * | <1 | - | 1 |
| Legende: c(Kat) = Katalysatorkonzentration (Metall) POZ = Peroxid Zahl | | | | | |

| | | | | | |
|---|---|---|---|---|---|
| *) 55 mg Pt/Aktivkohle (5 gew.-%ig) auf 300 g Tensidpaste (50 gew.-%ig). | | | | | |

## Patentansprüche

1. Verfahren zur Herstellung hellfarbiger Tenside mit vermindertem Peroxidgehalt, bei dem man Pasten anionischer, nichtionischer und/oder amphoterer bzw. zwitterionischer Tenside in Gegenwart von Platinmetallverbindungen mit Wasserstoffperoxid behandelt, **dadurch gekennzeichnet**, daß man als Platinmetallverbindung Palladium bzw. Platin auf Aktivkohle in Mengen von 10 bis 1000 ppm - bezogen auf den Feststoffgehalt der Paste - einsetzt.

2. Verfahren nach Anspruch 1, **dadurch gekennzeichnet**, daß man Tensidpasten mit einem Feststoffgehalt von 5 bis 85 Gew.-% einsetzt.

3. Verfahren nach den Ansprüchen 1 und 2, **dadurch gekennzeichnet**, daß man Tensidpasten anionischer Tenside einsetzt, die ausgewählt sind aus der Gruppe, die von Alkylbenzolsulfonaten, Alkansulfonaten, Paraffinsulfonaten, Olefinsulfonaten, Alkylethersulfonaten, Glycerinethersulfonaten, α-Methylestersulfonaten, Sulfofettsäuren, Alkylsulfaten, Fettalkoholethersulfaten, Glycerinethersulfaten, Monoglycerid(ether)sulfaten, Fettsäureamid-(ether)sulfaten, Hydroxymischethersulfaten, Sulfosuccinaten, Sulfosuccinamaten, Sulfotriglyceriden, Isethionaten, Tauriden, Sarcosinaten, Ethercarbonsäuren und Alkyl(ether)phosphaten gebildet wird.

4. Verfahren nach den Ansprüchen 1 bis 3, **dadurch gekennzeichnet**, daß man Tensidpasten nichtionischer Tenside einsetzt, die ausgewählt sind aus der Gruppe, die von Alkyloligoglykosiden, Saccharosestern, Sorbitanestern und Polysorbaten gebildet wird.

5. Verfahren nach den Ansprüchen 1 bis 4, **dadurch gekennzeichnet**, daß man Tensidpasten amphoterer bzw. zwitterionischer Tenide einsetzt, die ausgewählt sind aus der Gruppe, die von Alkylamidobetainen, Aminopropionaten, Imidazoliniumbetainen und Sulfobetainen gebildet wird.

6. Verfahren nach den Ansprüchen 1 bis 5, **dadurch gekennzeichnet**, daß man die Tensidpasten mit 0,1 bis 5 Gew.-% - bezogen auf den Feststoffgehalt der Paste - Wasserstoffperoxid behandelt.

## Claims

1. A process for the production of light-coloured surfactants of reduced peroxide content, in which pastes of anionic, nonionic and/or amphoteric or zwitterionic surfactants are treated with hydrogen peroxide in the presence of platinum metal compounds, characterized in that palladium or platinum on active carbon is used as the platinum metal compound in quantities of 10 to 1,000 ppm, based on the solids content of the paste.

2. A process as claimed in claim 1, characterized in that surfactant pastes having a solids content of 5 to 85% by weight are used.

3. A process as claimed in claims 1 and 2, characterized in that surfactant pastes of anionic surfactants selected from the group consisting of alkyl benzenesulfonates, alkane sulfonates, paraffin sulfonates, olefin sulfonates, alkyl ether sulfonates, glycerol ether sulfonates, α-methyl ester sulfonates, sulfofatty acids, alkyl sulfates, fatty alcohol ether sulfates, glycerol ether sulfates, monoglyceride (ether) sulfates, fatty acid amide (ether) sulfates, hydroxy mixed ether sulfates, sulfosuccinates, sulfosuccinamates, sulfotriglycerides, isethionates, taurides, sarcosinates, ether carboxylic acids and alkyl (ether) phosphates are used.

4. A process as claimed in claims 1 to 3, characterized in that surfactant pastes of nonionic surfactants selected from the group consisting of alkyl oligoglycosides, saccharose esters, sorbitan esters and polysorbates are used.

5. A process as claimed in claims 1 to 4, characterized in that surfactant pastes of amphoteric or zwitterionic surfactants selected from the group consisting of alkyl amidobetaines, aminopropionates, imidazolinium betaines and sulfobetaines are used.

6. A process as claimed in claims 1 to 5, characterized in that the surfactant pastes are treated with 0.1 to 5% by weight, based on the solids content of the paste, of hydrogen peroxide.

## Revendications

1. Procédé de préparation de tensioactifs de couleur claire à teneur en peroxyde réduite, dans lequel on traite des pâtes de tensioactifs anioniques, non ioniques et/ou amphotères ou zwitterioniques en présence de composés du groupe du platine avec du peroxyde d'hydrogène,
caractérisé en ce qu'
on utilise comme composé du groupe du platine du palladium ou du platine sur charbon actif en des quantités de 10 à 1000 ppm, par rapport à la teneur en solide de la pâte.

2. Procédé selon la revendication 1,
caractérisé en ce qu'
on utilise des pâtes de tensioactifs ayant une teneur en solide de 5 à 85 % en poids.

3. Procédé selon les revendications 1 et 2,
caractérisé en ce qu'
on utilise des pâtes de tensioactifs de tensioactifs anioniques qu'on choisit dans le groupe constitué des alkylbenzènesulfonates, des alcanesulfonates, des paraffinesulfonates, des oléfinesulfonates, des alkyléthersulfonates, des glycérinéthersulfonates, des a-méthylestersulfonates, des sulfoacides gras, des alkylsulfates, des éthersulfates d'alcool gras, des éthersulfates de glycérine, des (éther) sulfates de monoglycérides, des (éther) sulfates d'amide d'acide gras, des hydroxyéthersulfates mixtes, des sulfosuccinates, des sulfosuccinamates, des sulfotriglycérides, des iséthionates, des taurides, des sarcosinates, des acides éthercarboxyliques et des alkyl(éther)phosphates.

4. Procédé selon les revendications 1 à 3,
caractérisé en ce qu'
on utilise des pâtes de tensioactifs non-ioniques qu'on choisit dans le groupe des alkyloligoglycosides, des esters de saccharose, des esters de sorbitane et des polysorbates.

5. Procédé selon les revendications 1 à 4,
caractérisé en ce qu'
on utilise des pâtes de tensioactifs de tensioactifs amphotères ou zwitterioniques, qu'on choisit dans le groupe des alkylamidobétaïnes, des aminopropionates, des imidazoliniumbétaïnes et des sulfobétaïnes.

6. Procédé selon les revendications 1 à 5,
caractérisé en ce qu'
on traite les pâtes de tensioactifs avec 0,1 à 5 % en poids, par rapport à la teneur en solide de la pâte, de peroxyde d'hydrogène.
